(19) Europäisches Patentamt
European Patent Office
Office européen des brevets

(11) **EP 4 548 907 A1**

(12) **EUROPEAN PATENT APPLICATION**

(43) Date of publication:
07.05.2025 Bulletin 2025/19

(21) Application number: 23207262.9

(22) Date of filing: 01.11.2023

(51) International Patent Classification (IPC):
*A61K 8/19* (2006.01)        *A61K 8/44* (2006.01)
*A61Q 11/00* (2006.01)

(52) Cooperative Patent Classification (CPC):
A61Q 11/00; A61K 8/19; A61K 8/44

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: SkyLab AG
1066 Epalinges (CH)

(72) Inventors:
• Belous, Elena Yur'evna
121309 MOSCOW (RU)
• Ivanova, Angelina Dmitrievna
London, NW61NE (GB)

(74) Representative: Glawe, Delfs, Moll
Partnerschaft mbB
Postfach 13 03 91
20103 Hamburg (DE)

(54) **A SYSTEM FOR RESTORING HARD TISSUES OF THE TOOTH BASED ON ASPARTIC ACID THAT CREATES CRYSTALLISATION NODES AND BINDS CALCIUM TO INCREASE THE TOOTH MICROHARDNESS**

(57) The invention relates to a composition comprising the specific combination of aspartic acid and a calcium source, wherein the aspartic acid is present in a specific wt.% range of 0.05 wt.% to 1.00 wt.%. Said composition enables biomimetic remineralization of hard tooth tissues, including enamel, dentin, cement and can be used, inter alia, in oral care products.

Visualization of the molecular structure of aspartic acid and asparagine demonstrating reaction groups

**Fig. 1**

**Description**

TECHNICAL FIELD

The present invention relates to a pH-stable $\alpha$-amino acid technology - aspartic acid of the formula $C_4H_7NO_4$. Use of aspartic acid enables biomimetic remineralization of hard tooth tissues (enamel, dentin, cement) in pH ranges of oral care products by binding calcium and creating crystallization nodes for growth of hydroxyapatite of the formula $Ca_{10}(PO_4)_6(OH)_2$ (a mineral from the apatite group being the basic material of hard tooth tissues), thus increasing its microhardness.

Use of the technology will make it possible to restore damaged enamel on the level of and/or above fluorine, the benchmark recognized on the market, thus being a preventative, restoring and desensitive means in case of destruction of hard tooth tissues under the impact of external (whitening, eating/drinking of acid/alkaline/hot/cold food/beverages, smoking, and other reasons for destruction of hard tooth tissues) or internal factors.

THE PRIOR ART AND THE GIST THE INVENTION AS DEFINED IN THE CLAIMS

One of the problems connected with oral cavity is dental hyperesthesia - increased sensitivity of hard tooth tissues taking the form of short-term pain arising in response to action of different stimuli (chemical, thermal or tactile ones). As such, pain accompanies eating and drinking, tooth brushing, etc. Saliva pH reduction results in dissolution of tooth enamel apatite crystals, which causes enamel demineralization. Enamel weakens, exposing dentin and its tubules. Then pathogenic microflora starts to penetrate inside the tooth, gradually destroying dentin and affecting pulp. The demineralization process can also take place in absence of different pathologies, for example, when eating food with a high amount of carbohydrates (pastry, sweet food, etc.), excessive drinking of alcohol, coffee and tea as well as beverages with a high content of sugar. The cause is saliva pH reduction during consumption of these products [1]. More acidic saliva pH contributes not only to enamel destruction, but to increasing of the quantity of bacteria in oral cavity as well. When the quantity of pathogenic bacteria in oral cavity increases, the risk of development of oral cavity diseases, especially, caries and periodontitis, is high. They are the most widely spread diseases based on changes in the composition of the normal microbiological community, but not integration of pathogenic microorganisms from the environment [2]. Enamel destruction and resultant dentin exposure can cause different diseases as it is the main reason for tooth hypersensitivity, caries, etc. As a result, at present there is a need in development of oral care products that will not only effectively clean oral cavity and increase pH, preventing further growth of pathogenic bacteria and enamel and dentin destruction, but will also restore hard tooth tissues during tooth brushing.

The tooth consists of mineralized and non-mineralized tissues. The first ones include enamel, dentin and cement. Non-organic components prevail and organic compounds and water are present in their chemical composition. All these tissues consist of the intercellular substance, or matrix, having carbohydrate-protein nature and a high quantity of mineral substances, mainly represented by apatite crystals. The mineralization degree is expressed in the following way: enamel, dentin, cement.

There is one soft (non-mineralized) tissue in the tooth, it is called pulp and is located in the tooth crown and root cavity.

Enamel covering the tooth crown is the hardest tissue in the organism, which is explained by a high concentration of non-organic substances therein (up to 97%), mainly, crystals of apatites: hydroxyapatite (up to 75%), carbonate apatite, fluorapatite, chloroapatite, etc. The main function of enamel is protecting dentin and pulp against external mechanical, chemical and thermal stimuli. The smallest structural units of enamel are crystals of apatite-like substance forming enamel prisms. The mineral basis is composed of apatite crystals and Ca8H2(PO4)6*5H2O phosphate; the formula of basic hydroxyapatite is Ca10(PO4)6(OH)2. Hydroxyapatite has a hexagonal form. The long axis of prism crystals is located along the main direction of pressure on bone or tooth. Each crystal is covered with a hydration coating of about 1 nm. Bound water forming this coating makes up about 3.0-3.3% of enamel weight.
Presence of vacant places in hydroxyapatite crystalline lattice is an important factor in activation of isomorphous substitutions inside the crystal. The hydroxyapatite crystal composition and properties constantly change and depend on ionic composition of the liquid that washes the crystal as well as on the hydration coating composition. These properties of crystals make it possible to change the composition of hard tooth tissues in a targeted manner, using remineralizing solutions with the aim of caries prevention or treatment.
The basis for enamel formation and functional construction is its protein matrix the elementary functional unit of which is calcium-binding protein of enamel (CBPE). The latter is capable of oligo- and polymerization by means of calcium bridges with formation of a three-dimensional protein network insoluble in neutral medium.

Dentin is located under enamel and makes up the main hard tooth part. The main dentin substance consists of hydroxyapatite. However, in distinction to enamel, a large quantity of dentin tubules run through dentin. Pain sensations are transferred over nerve receptors. Dentin also contains a large, comparing to enamel, quantity of organic molecules.

Dentin protein matrix is 20 % of the total dentin weight. It consists of collagen, its share is 35 % of all organic dentin substances. Collagen has a special structure, it does not swell in water, it is resistant to the impact of collagenase, it contains up to 12% of hydroxyproline, 2.0-3.5% of lysine and hydroxylysine. It also contains a large quantity of glutamic and aspartic acids, arginine, leucine, isoleucine and valine, few cyclic amino acids and no tryptophan.

Dentin also contains 1% of citric acid the role of which consists in the ability of forming chelate complexes with calcium for its transmission into hydroxyapatite. In addition, the dentin's own protein is calcium-binding protein.

Remineralization is the natural response of the organism to demineralization caused by different factors. During demineralization ions exit hydroxyapatite crystals and vacant places are formed in the crystal where the same or similar ions can enter.

On all stages of ionic substitution the critical factors controlling the ion exchange rate and degree are the following:

- concentration of $Ca^{2+}$, $PO^{3-}$, F-4 ions of the medium surrounding the crystal;
- duration of their impact;
- physical and chemical properties of these ions;
- charge on the surface and in the depth of crystals;
- pH of the medium surrounding the crystal.

Remineralization is a partial change or complete restoration of mineral tooth enamel components by components of saliva (or other solutions). Remineralization is based on adsorption of mineral substances into carious areas and submersion of ions in vacant places left by ions that exited the hydroxyapatite crystal. They can be substituted by the same or similar ions. Properties of hydroxyapatite crystals can change in case of substitution for ions of another kind. For example, substitution of a calcium ion with another ion in hydroxyapatite can be accompanied with:

- reduction of resistance of crystals to unfavorable impacts of chemical and physical factors;
- crystal deformation and fragility.

A criterion of effectiveness of remineralizing solutions is such properties of enamel as its permeability and solubility, disappearance or reduction of a carious spot, greater caries growth. The main part of organic and mineral substances that are transferred to tooth enamel are contained in saliva. Saliva is known to cause swelling or shrinking of collagen barriers by acting on tooth enamel. As a result enamel permeability changes. Saliva substances exchange with enamel substances and de- and remineralization processes are based thereon. Enamel is easily permeable for $H_2O$, ions (phosphates, bicarbonates, chlorides, fluorides, cations of Ca, Mg, K, Na, F, Ag, etc.). They determine the normal tooth enamel composition. However, ion exchange between saliva and enamel cannot take place too quickly, diffusion of substances is limited by rate for maintaining balance because if the diffusion rate is too high, components will leave enamel and the tooth will be destroyed faster as well. In this case if a person consumes sweet food and beverages, tea, coffee, alcohol, etc. often, the natural remineralization system will not cope with it and it is necessary to use additional remineralizing oral care products to maintain tooth health.

Principles of minimal invasive dentistry clearly state the need in clinically effective measures for remineralization of early tooth destruction.

While fluorine-mediated remineralization is a cornerstone of modern remineralization philosophy, a number of new remineralization strategies have been commercialized or are at the stage of development which are said to contribute to deeper remineralization of lesions, reduce potential risks connected with oral care products with a high content of fluoride and simplify control over enamel destruction throughout life. These non-remineralizing systems can be divided into biomimetic regenerative technologies and approaches that restore enamel by means of fluoride or systems improving fluoride effectiveness.

The key discovery of fluoride as an agent that can prevent tooth caries became one of the most important landmarks in dentistry [3]. The mass of fluoride in human organism is about 7 mg; biotic compounds are concentrated in bone tissue, teeth and nails. Almost 99% of all the fluoride in the organism are located in hard tissues in the form of fluorapatites. Small crystals submerged in organic matrix prevail in the mineral phase. Fluoride-ion surpasses all other ions by its ability to substitute -OH thanks to closeness of their ionic radii, equal charges and the hydration degree equal to two. Fluoride is included in apatite either in the period of formation of the primary crystal or by substitution of -OH in the transformed crystal. As a result of the substitution reaction a mixed form of apatite of the formula $Ca_{10}(PO_4)_6(OH)_2-XF_X$ is created. The content of this element in bone and tooth enamel is usually about 0.05 mol/kg and demonstrates - OH-fluoride ratio in the apatite molecule of 40: 1. The first place by the content of fluoride among hard tissues belongs to tooth cement, it is followed by bone, dentin and enamel. Fluoride plays a significant role not only in initial stages of hard tissue mineralization, but also prevents their demineralization. Fluorine makes fluorapatite crystals more organized, thus reducing their solubility at physiological pH value. The biological action of the fluorine ion is based on its ability to effectively substitute the hydroxide-ion not only in bone tissue hydroxyapatite, but in non-mineralized tissues as well. An obvious approach to increasing the remineralization potential of fluorine would be simple addition of a larger quantity of fluorine in oral care products as fluorine shortage can lead to osteoporosis and caries, however, fluorine excess results in fluorine intoxication and manifests itself in oral cavity as dental fluorosis. Also, bioavailability of $Ca^{2+}$ and $PO_4^{3-}$ ions (being the basic construction material for hydroxyapatite) is often a limiting factor for general remineralization in case of local application of fluorides, and it is especially aggravated in hyposalivation conditions [4, 5]

In bones and teeth of an adult person about 1 kg of the element is in the form of hydroxyapatite the main elements of which are calcium and phosphorus. Calcium belongs to macroelements. Its total quantity in the organism is 1.4% of the body weight. Calcium is contained in each cell of the human organism. Mainly, this element is located in bone and dental tissues. On average, an adult person should take 1 g of calcium daily, although the need therein is only 0.5 g. This is connected with the fact that calcium taken with food is absorbed in the intestine only by 50% as it forms low-solubility compounds (calcium phosphate and calcium salts of fatty acids) in the gastrointestinal tract.

By the content in the human organism (0.95%) phosphorus belongs to macroelements. Phosphorus is an organogen element and plays an extremely important role in metabolism. Phosphorus in the form of phosphates is a part of proteins (phosphoproteins) - 0.5-0.6% of the total quantity of nucleic acids, mono- and dinucleotides, phospholipids and a number of other compounds. Phosphorus is the basis of the skeleton (calcium orthophosphate, hydroxyapatite) and teeth (hydroxyapatite, fluorapatite, etc.).

Natural remineralization takes place due to buffer properties of saliva which contains phosphates and calcium that induce formation of hydroxyapatite crystals.

Oral care products containing fluorides and calcium sources are effective for enamel remineralization but cannot contribute to formation of organized apatite crystals [6, 7].

At present, an attempt is made to shift from reparative to regenerative biomineralization therapy when impaired tooth tissues are replaced with biologically similar tissues [9]. However, enamel regeneration is an especially complex task as mature enamel is acellular and is not resorbed and not remodeled in distinction to bone or dentin [10]. Achievements in the field of tissue engineering methods led to biomimetic methods that demonstrated a great potential for regeneration of the hierarchical enamel microstructure which is proteins and peptides.

Among proteins of hard tooth tissues there is a number of proteins and peptides that take part in remineralization processes. Presence of a large quantity of glutamic and aspartic acids in these proteins enables binding of calcium directly to the carboxyl group of these amino acids. Thus, these proteins bind calcium to hydroxyapatite, the crystal is restored and remineralization takes place. Let's consider some of these proteins and peptides:

1. Osteonectin is a glycoprotein. Residues of glutamic (Glu) and aspartic (Asp) acids, amino acid and phosphorylated residues of lysine (Lys) bind $Ca^{2+}$ ions and connect to hydroxyapatites. The ability of binding $Ca^{2+}$ ions, collagen of type I and HAP enables osteonectin to form crystallization centers and initiate the bone tissue mineralization process.

2. Osteopontin is an acidic glycoprotein synthetized by osteoblasts. It contains aspartic acid repeats that bind $Ca^{2+}$ ions and hydroxyapatite crystals.

3. Amelogenins, enamelins and taftelins are enamel proteins controlling the remineralization process, growth of hydroxyapatites, etc. Functions of each individual protein are not fully understood yet, but as a complex they take direct part in initiating and supporting the remineralization process.

In cases when the organism does not cope with tooth remineralization by itself, the consumer has a need in using external additional products with remineralization agents. The authors studied components that take part in natural remineralization, and it is suggested to use aspartic acid as the remineralizing component.

Aspartic acid is an available and inexpensive component that is suitable for inclusion in different formulations and will remain stable in products.

In the course of work the authors discovered a number of researches devoted to use of polyaspartic acid and complexes with its inclusion for tooth remineralization [11, 12, 13, 14]. However, despite successful study results, polyaspartic acid is not easily available and is expensive, owing to which it will be hard to make the final product on the industrial scale and it will not be within an affordable price category.

There is a similar situation with proteins that take part in remineralization: amelogenins, enamelins, etc. There were found researches on testing products with some enamel proteins. [15, 16]. They are not commercially available and not of a low price category, which impedes their use in a product as additional chemical synthesis is required, which will increase the price of the final product on the market and make it less affordable for wide consumer use; in addition, they are not stable outside live organisms, which also impedes their commercial use in a product owing to problems with additional stabilization in a product.

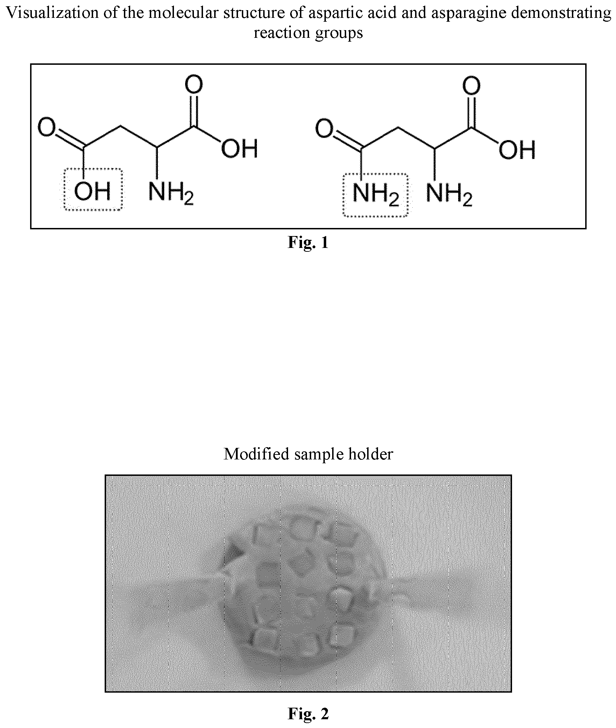

Aspartic acid is a negatively charged polar amino acid. It represents a colorless crystalline substance, it melts with decomposition at 270$^0$C. Aspartic acid has low solubility in water and is almost insoluble in alcohol and ether. The structure of aspartic acid is shown in Fig 1.

By means of two reactive carboxyl groups aspartic acid can bind calcium ions and, first of all, bind calcium with hydroxyapatite and, secondly, form crystallization nodes on tooth enamel, initiating remineralization. [17, 18]

Also experiments carried out by the authors prove the remineralization ability of aspartic acid without catalyzers and additives at lower concentrations from 0.05% to 0.2% by means of saliva resources.

Apart from the unexpected result obtained by the authors with respect to aspartic acid they also discovered the synergistic effect between aspartic acid in a higher concentration (from 0.2% to 1%) and a calcium source, as a result of which the effect of remineralization and increasing of microhardness of hard tooth tissues surpasses the results of fluorine.

Thus, the invention generally relates to aspartic acid and its use in oral care products that enables achieving such results as effective enamel remineralization and increasing of its microhardness by means of resources present in saliva, and the results surpass by effectiveness those of fluorine in combination with calcium-containing substances without consequences of use of fluorine-containing systems in the form of fluorosis.

The main problem of implementation of mineralizing prevention is instability of solutions with ions of calcium, phosphates and fluoride (fast combination of ions into molecules, reduction of bioavailability of minerals), therefore conventionally applications of preparations with fluoride, calcium and phosphate are offered. The most popular combinations of monopreparations are applications with 10 % calcium gluconate solution, 2.5 % calcium glycerophosphate solution or 5 % calcium chloride solution. [17]

Remineralization takes place due to diffusion of soluble bioavailable ions of calcium and phosphate from outside through nanoporosity of the surface enamel layer into the subsurface lesion and due to deposition of these ions in the cavity of apatite crystals inside the lesion. Although saliva is an important source of ions for remineralization, in itself it has a relatively low potential for remineralization of carious tissue of the tooth.

Anticariogenic fluorides are powerful factors of remineralization, contributing to reduction of incidence of caries in the whole world in the twentieth century from the moment of their appearance. However, remineralization promoted by fluorides takes place primarily in the surface area of carious lesions and is limited by bioavailability of ions of calcium and phosphate present in saliva and dental plaque. [18]

Fluorine ions contribute to formation of fluorapatite in enamel in presence of ions of calcium and phosphate that form during demineralization of enamel by organic acids of dental plaque bacteria. At present it is believed that this is the main mechanism of action of fluorine ions in prevention of enamel demineralization. Fluorine ions can also promote remineralization of earlier demineralized enamel if there is a sufficient quantity of ions of calcium and phosphate in saliva or dental plaque during use of fluorine. Non-invasive treatment of early caries by means of remineralization can become an important step forward in clinical treatment of this disease. However, 10 calcium ions and six phosphate ions are required per each two fluorine ions to form one elementary cell of fluorapatite ($Ca_{10}(PO_4)_6F_2$). As a result, in case of local use of fluorine ions availability of calcium and phosphate ions can be a limiting factor for pure remineralization of enamel, which is strongly aggravated in conditions of, for example, xerostomia.

Clinical use of calcium and phosphate ions for remineralization in the past was not successful because of low solubility of calcium phosphates, especially in presence of fluorine ions. Insoluble calcium phosphates are not easily applied, are not localized effectively on the tooth surface and require acid solubility for formation of ions capable of diffusing to subsurface enamel lesions. On the other hand, soluble calcium and phosphate ios can be used only in very low concentrations due to insolubility characteristic for calcium phosphates, in particular, calcium fluoride phosphates. Soluble calcium and phosphate ions are almost not included in dental plaque and are not localized on the tooth surface to create effective concentration gradients enabling diffusion to subsurface enamel. [19]

ESSENCE OF INVENTION

The invention relates to the use of the aspartic acid according to the invention in an oral care products, as toothpastes, mouthwashes, oral foams, gels, strips, for teeth remineralization and enamel restoration. The oral care products according to the invention may contain 0.05 to 1.00 wt.%.

It also can contain calcium sources with crystallin or amorphous structure and particle sizes from 0,001 $\mu$m to 100 $\mu$m in concentration 0.1 to 15.00 wt.%, which can be soluble or insoluble in water.

For example: hydroxyapatite, calcium hydroxyapatite, calcium/magnesium/zinc hydroxyapatite, zinc hydroxyapatite, aluminum/calcium/zinc hydroxyapatite, calcium/strontium hydroxyapatite, calcium chitosan, calcium sodium fluorophosphosilicate, calcium acrylate, calcium citrate, calcium sorbate. calcium pantothenat, calcium gluconate, calcium aluminum borosilicate, calcium sodium phosphosilicate, calcium polygamma-glutamate, calcium carbonate, calcium monofluorophosphate, calcium silicate, calcium oxide, calcium ascorbate, calcium glycerophosphate, calcium peroxide, calcium lactate, dicalcium phosphate dihydrate, dicalcium phosphate, calcite, hydroxyapatite (nano), potassium calcium hydroxyapatite, octacalcium phosphate, amorphous calcium phosphate, $\alpha$, tricalciumphosphate, $\beta$-tricalcium-phosphate, dicalcium phosphate anhydrate, monocalcium phosphate monohydrate, monocalcium phosphate anhydrate.

EXPERIMENTAL SECTION

The examples included in the present description are not intended to limit the claimed invention and are merely intended to illustrate and confirm the achievement of the expected technical results. These examples are among the many experimental data obtained by the authors of the invention which confirm the effectiveness of the means within the scope of the invention. With regard to the examples of the invention given below, it should be noted that a aspartic acid having the CAS number CAS 56-84-8 / 617-45-8 was used.

EXPERIMENT 1

An *In Vitro* Study to Compare the Ability of Calcium Sources Versus a Positive and Negative Control to Repair and Protect Demineralised Samples.

1. Study Overview

This *in vitro* study was designed to compare the remineralization efficacy of the test products, following a single, overnight treatment application.

Bovine enamel blocks were sectioned, machine polished and flattened. The baseline surface microhardness of the enamel samples was measured with a Buehler MicroMet 5103 surface microhardness machine.

Each bovine enamel block was demineralised for 1 hour by immersing the sample at 37°C, in a demineralization solution described by Huang *et. al.* (2009)[1]. Following demineralization, the surface microhardness of each enamel sample was remeasured.

All demineralized enamel samples were subjected to an overnight immersion in the assigned test product, with immersion solutions mixed and heated to 37°C during treatment. Following the overnight immersion, the surface microhardness of the enamel samples was remeasured. The study logistics were designed so that each enamel sample was demineralized and then immersed in the assigned treatment on the same day.

The ability of a single, overnight application of test products and controls to repair enamel lesions was assessed by comparing the percentage surface microhardness recoveries and the changes in surface microhardness for the treated samples.

1.1 Test Products

Table 1. Test Products

| Treatment Group | Treatment | Solution Preparation |
|---|---|---|
| 1 | nanoXim* CarePaste Hydroxyapatite (nano) 15% solution Prepared as a 1% solution in deionised water | 2.70g nanoXim*CarePaste Hydroxyapatite (nano) 15% solution made up to 40.01g with deionised water pH adjusted to 7.17 with 1% HCl |
| 2 | CaMgZnAHAp 10% - EUXYL solution Prepared as a 1% solution in deionised water | 4.02g CaMgZnAHAp 10%-EUXYL made up to 40.12g with deionised water pH adjusted to 7.26 with 1% HCl |
| 3 | CaHAP-Dry 100% dry powder Prepared as a 1% solution in deionised water | 0.41g CaHAP-Dry made up to 40.16g with deionised water pH adjusted to 7.05 with 1% HCl |
| 4 | CaMgZnAHAP-Dry 100% dry powder Prepared as a 1% solution in deionised water | 0.41g CaMgZnAHAP-Dry made up to 40.32g with deionised water pH adjusted to 7.07 with 1% HCl |
| 5 | CaHAP 15%-SMFP solution Prepared as a 1% solution in deionised water | 2.65g CaHAP15% made up to 40.07g with deionised water pH 7.24 (Not adjusted) |
| 6 | OCP 100% dry powder Prepared as a 1% solution in deionised water | 0.40g OCP made up to 40.15g with deionised water pH 6.99 (Not adjusted) |
| 7 | Tricalcium Phosphate 100% dry powder Prepared as a 1% solution in deionised water | 0.40g Tricalcium Phosphate made up to 40.29g with deionised water pH 7.02 (Not adjusted) |

(continued)

| Treatment Group | Treatment | Solution Preparation |
|---|---|---|
| 8 | Fluoride (Positive control) 100% Sodium Fluoride powder Prepared as 1450ppm F in deionised water | 0.3238g of NaF powder made up to 100.04g with deionised water. pH 7.17 (Not adjusted) |
| 9 | Deionised water (Negative control) SLS Code: CHE3876 | Used as supplied by manufacturer. |

1.2 Block Preparation

One hundred and thirty enamel blocks, measuring 4 x 4 mm, were cut from bovine incisors and lapped planar-parallel using a Logitech PM5 lapping machine. The enamel surfaces of the samples were machine-polished using a MetPrep Saphir 550 to a final finish of 0.04 microns. One corner of each block was removed in order to ensure correct orientation on the SMH machine.

The enamel samples were sonicated, and surface checks performed with an objective lens of the Buehler MicroMet 5103 microhardness indenter. Suitable enamel samples were stored refrigerated, in individual 7 ml Sterilins, in deionised water.

1.3 Baseline SMH Measurements

The baseline surface microhardness of the enamel samples was measured using a calibrated Buehler MicroMet 5103 microhardness indenter and Knoop diamond indenter tip.

The enamel samples were orientated with the abraded corner in the top right. Baseline surface microhardness indents were placed in the centre of the enamel samples. Five Knoop indents were placed in a vertical line at each timepoint (under a load of 50 g for 10 seconds). Surface imperfections were avoided, and outlying indents replaced.

Each enamel block was required to have an average baseline Knoop SMH > 250.0 HK (Hardness Knoop) and a standard deviation of < 20.0 in order to progress to the demineralization phase of the study.

1.4 Removal of Outliers and Stratification

Specimens with 'outlier' baseline SMH values (<250) or standard deviations (> 20.0) were removed from the study. 90 were stratified into 9 treatment groups (n = 10) to create groups with similar mean baseline surface microhardness values.

Note: Three samples didn't meet these criteria, but were used in the study, and behaved similarly to other samples.

1.5 Initial Enamel Block Demineralization

A demineralization solution was prepared according to the following specifications:

- 50 mM Acetic acid
- 2.2 mM Calcium nitrate
- 2.2 mM Potassium phosphate monobasic
- 0.1 ppm Sodium fluoride
- Final pH of the solution was adjusted to 4.5 with sodium hydroxide.

Enamel samples were mounted onto acetate squares using double-sided tape. Enamel samples were immersed in 8 ml demineralization solution per sample and placed into an oven for 1 hour (37 °C). The enamel samples were then removed from the demineralization solution, rinsed with deionized water for 2 minutes and returned to their original containers.

1.6 Post-demineralization SMH Measurements

The post-demineralization surface microhardness of the enamel samples was measured using a calibrated Buehler MicroMet 5103 microhardness indenter and Knoop diamond indenter tip.

The enamel samples were orientated with the abraded corner in the top right. Post-demineralization Knoop surface microhardness indents were placed approximately 100 microns from the baseline indents. Five Knoop indents were placed in a vertical line at each timepoint (under a load of 50 g for 10 seconds). Surface imperfections were avoided, and outlying indentations replaced.

2. Main study

2.1 Treatment

The enamel samples in each treatment group were attached to a modified sample holder using Aquasil Hard Putty (Fig. 2). Ten enamel samples were attached to a modified post for each of the treatment groups (Table 1).

The enamel samples in each treatment group were treated with a single, overnight treatment with assigned test product for 16 hours. The aim of this exaggerated treatment regime was to accentuate any possible mineralizing effects of the test products.

All stages of treatment were carried out in an incubator at 37° C on a magnetic stirrer.

2.2 Post-treatment (SMH) Measurements

The post-treatment surface microhardness of the enamel samples was measured with a calibrated Buehler MicroMet 5103 microhardness indenter and Knoop diamond indenter tip.

The enamel samples were orientated with the abraded corner in the top right. Post-treatment Knoop surface microhardness indents were placed approximately 100 microns from the baseline indents. Five Knoop indents were placed in a vertical line at each timepoint (under a load of 50 g for 10 seconds). Surface imperfections were avoided, and outlying indentations replaced.

2.3 Data Management and Statistical Analysis

The following formulae were used to assess the remineralization efficacy of the test products:

Percentage SMH recovery (%SMHR) was calculated as:

- 

$$\% \ SMHR = 100 * [(SMH \ Post \ Treatment - SMH \ Post\text{-}demineralization) / (SMH \ Baseline - SMH \ Post\text{-}demineralization)].$$

Surface microhardness change (SMHC)

- Post-treatment SMH - Demineralisation SMH

Minitab18 software was used to calculate descriptive statistics for the %SMHR and the SMHC increases achieved by each test product (Mean, median, maximum, minimum, standard deviation).

The %SMHR values and SMHC for enamel lesions treated by the test products was statistically compared using a General Linear Model ANOVA. A Tukey test was selected to make pairwise statistical comparisons.

3. Results

3.1 Percentage Surface Microhardness Recovery (%SMHR) Data

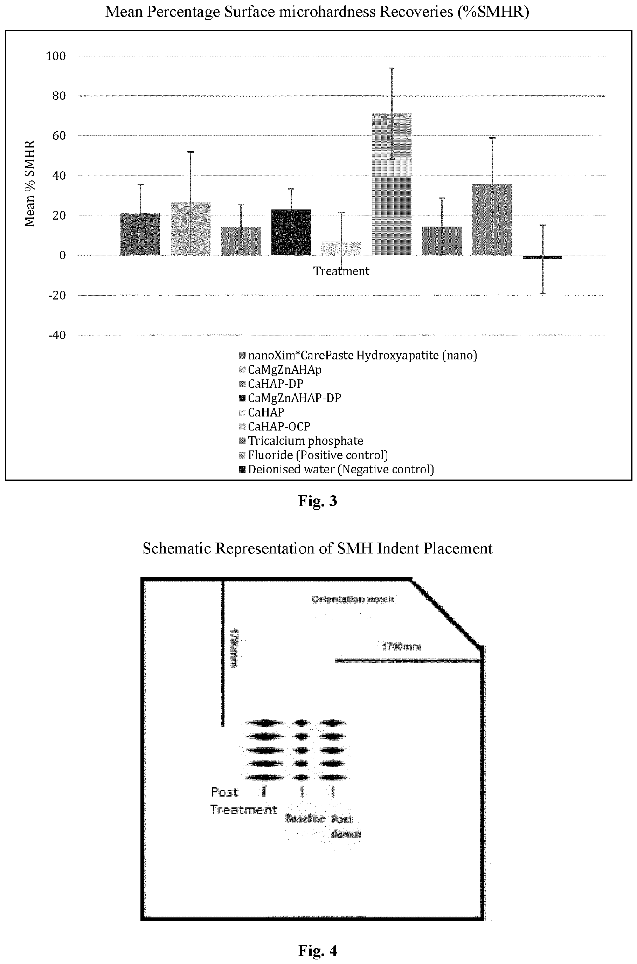

This part of the results section will discuss the percentage surface microhardness recoveries (%SMHR) for enamel lesions after exposure to a single, overnight immersion in the assigned treatments. The exaggerated treatment regime was selected to accentuate any possible mineralizing effects of the test products. Higher %SMHR values are indicative of greater remineralization. The %SMHR data is presented in Table 2 and displayed graphically in Figure 3.

Table 2. Mean Percentage Surface Microhardness Recoveries (%SMHR)

| Treatment | Mean (%SMR) | Grouping | | | | |
|---|---|---|---|---|---|---|
| 1% OCP | 71.1946 | A | | | | |
| 1450 ppm F | 35.5556 | | B | | | |
| 1% CaMgZnAHAp | 26.6809 | | B | | | |
| 1% CaMgZnAHAP-Dry | 22.9939 | | B | | | |
| 1% NanoXim*CarePaste Hydroxyapatite (nano) | 21.3436 | | B | C | | |
| 1% Tricalcium phosphate | 14.4983 | | | C | | |
| 1% CaHAP-Dry | 14.3123 | | | C | D | |
| 1% CaHAP | 7.2579 | | | C | D | |
| Deionised water | -1.8685 | | | | | E |
| *Means that do not share a letter are significantly different. | | | | | | |

The highest %SMHR value was achieved by the 1% OCP test product (+71% SMHR), which achieved a statistically significantly higher %SMHR value than all comparator test products.

The second highest %SMHR value was achieved by the 1450 ppm Fluoride control (+36% SMHR), which was statistically significantly higher than the %SMHR values achieved by 1% CaHAP and deionised water.

The lowest %SMHR values were achieved by deionised water (-2 %SMHR). These test products were the only treatments to cause negative %SMHR changes and achieved statistically significantly lower levels of %SMHR values than 1% OCP, 1450 ppm Fluoride and 1% CaMgZnAHAp.

3.2 Change in Enamel Surface Microhardness (SMHC) Data

This part of the results section will discuss the post treatment increases in enamel surface microhardness hardness achieved by each test product. The change in SMHC data differs from the %SMHR data in that it only assesses the change in the surface microhardness of the enamel lesions after treatment and ignores the original hardness of the samples before they were demineralised. The change in SMH data is shown in Table 3 and presented visually in Figure 4.

Table 3. Mean Post Treatment Change in the Lesion Surface Microhardness (SMH)

| Treatment | Mean Change in SMH | Grouping | | | |
|---|---|---|---|---|---|
| 1% OCP | 40.76 | A | | | |
| 1450 ppm FLuoride | 25.12 | A | B | | |
| 1% CaMgZnAHAp | 24.26 | A | B | | |
| 1% CaMgZnAHAP-Dry | 22.20 | A | B | | |
| 1% NanoXim*CarePaste Hydroxyapatite (nano) | 18.52 | | B | | |
| 1% CaHAP-Dry | 13.50 | | B | C | |
| 1% Tricalcium phosphate | 9.82 | | B | C | D |
| 1% CaHAP | 6.16 | | B | C | D |
| Deionised water | -3.00 | | | C | D |
| Means that do not share a letter are significantly different. | | | | | |

The most effective test product at increasing the mean SMH of the enamel samples was 1% OCP, which achieved a mean SMH increase of +41 HK units. Statistical comparison of the data showed the 1% OCP achieved a directionally higher mean SMH increase than 1450 ppm Fluoride, 1% CaMgZnAHAp and 1% CaMgZnAHAP-dry. The 1% OCP test product achieved statistically a significantly higher mean increase in the SMH of the enamel lesions than 1% NanoXim*CarePaste Hydroxyapatite (nano), 1% Tricalcium phosphate, 1% CaHAP, Deionised water.

The next best performing test products at increasing the mean SMH of the enamel lesions was 1450 ppm Fluoride (+ 25 HK units), followed by 1% CaMgZnAHAp (+ 24 HK units), and 1% CaMgZnAHAP-dry (+ 22 HK units). The 3 test products achieved a directionally higher mean increase in SMH than; 1% NanoXim*CarePaste Hydroxyapatite (nano), 1% CaHAP-dry, 1% Tricalcium phosphate and 1% CaHAP. The 3 test products achieved a statistically significant mean increase in SMH than deionised water.

The worst performing test product at increasing the mean SMH of the enamel lesions was deionised water (-3 HK units). Both test products achieved negative mean SMH changes in the hardness of the enamel lesions and were statistically significantly inferior to the following test products at increasing the mean SMH of the enamel samples; 1% OCP, 1450 ppm Fluoride, 1% CaMgZnAHAp, 1% CaMgZnAHAP-Dry and 1% NanoXim*CarePaste Hydroxyapatite (nano).

4. Conclusions

The best performing test product at repairing the enamel lesions was 1% OCP.
The next best performing test product at repairing the enamel lesions was 1450 ppm Fluoride, followed by 1% CaMgZnAHAp and 1% CaMgZnAHAP-Dry.
There was evidence of the 1450ppm fluoride control causing greater surface changes in the enamel lesions compared to the comparator test products. The greater surface changes may indicate fluorosis of the enamel lesions, which may be impacting upon the ability of the positive control to repair the enamel lesions.
The worst performing test products at repairing and protecting the enamel lesions were deionised water, which were the only test products to achieve negative remineralization values.
All test products with the exception of deionised water had a positive remineralizing effect.

EXPERIMENT 2

1. Study Design

Enamel blocks were sectioned from bovine teeth, lapped plano-parallel and highly polished. The baseline surface microhardness of the enamel samples was then measured.
The enamel samples were artificially demineralised by immersing them in demineralisation solution for 60 minutes. Following demineralisation, the surface microhardness of the enamel samples was remeasured. The samples were then stratified between the treatment groups based upon the baseline surface microhardness.
The enamel samples in each treatment group were treated with the assigned test products for 16 hours. Following treatment, the surface microhardness of each treated enamel sample was remeasured.
The percentage of surface microhardness recovery (%SMHR) achieved by the test products was calculated and used to compare the ability of the test products to repair the demineralised enamel samples.

1.1 Test Products

The test products detailed in Table 4 were assessed in this study.

Table 4. Test Product Codes

| Test Product Decryption | Test Product |
|---|---|
| L-Aspartic Acid | Barentz Batch: 1007 040 902 |
| Tricalcium Phosphate | No Batch ID. |
| Peptide | GL Biochem Shanghai Ltd, Sequence: DRNLGDSLHRQEI Lot: P220317-CQ410801 |
| Deionised Water | Deionised water (Negative control) SLS Code: CHE3786 Lot: 846747 |
| Sodium Fluoride | Sodium Flouride Sigma Aldrich, Code: 201154-100G Lot: MKCN1670 5th April 2027 |

The test products detailed in Table 4 were applied to the samples in the concentrations detailed in Table 5.

Table 5. Test Product Concentrations

| Treatment Group | Treatment |
|---|---|
| 1 | Aspartic Acid 0.5 %, pH 6-6.5 |
| 2 | Aspartic Acid 0.5 %, + Tricalcium Phosphate 1.0 %, pH 6-6.5 |
| 3 | Fluoride (Positive control) 100% Sodium Fluoride powder Prepared as 1450 ppm F in deionised water |
| 4 | Deionised water (Negative control) |
| 5 | Aspartic Acid 0.5 %, + Tricalcium Phosphate 1.0 %, Peptide 0.05 % pH 6-6.5 |
| 6 | Aspartic Acid 0.1 %, pH 6-6.5 |
| 7 | Aspartic Acid 0.1 %, + Tricalcium Phosphate 0.5 %, pH 6-6.5 |

The pH of each solution was adjusted into the pH range shown in Table 2, using sodium hydroxide or dilute hydrochloric acid. All pH measurements were taken with a calibrated pH electrode (Serial Number: 13-620-631) and either an Accumet XI,250 ion meter (Asset Number: UK01200-LAB-15-002), or an Orion 720 ion meter (Asset Number: 4F064).

1.2 Block Preparation

Approximately 90 enamel blocks (Measuring 4mm x 4mm) were sectioned from bovine incisors and were lapped plano-parallel (both sides) using a Logitech PM5 lapping machine (Asset number: 4F008).

The enamel surfaces of the samples were machine-polished with 1 micron and 0.3 micron aluminium oxide slurries, using a MetPrep Saphir 550 Polisher (Asset Number: UK01200-1010015-114).

One corner of each block was removed to facilitate consistent orientation of the samples on the surface microhardness machine. All prepared blocks were stored in individual Sterilins, between layers of tissue paper dampened with 0.1% thymol.

1.3 Surface Microhardness (SMH) Measurements

The surface microhardness of each block was measured at the following time points:

- At baseline.
- After the demineralisation treatment.
- After overnight treatment.

The surface microhardness of the enamel samples was measured using a Buehler MicroMet 5103 (Asset Number: 4F101) and OmniMet MHT software.

The Intertek CRS method for measuring the surface microhardness of the samples at each time point has been provided below:

- Five Knoop indents
- 50 gram load force
- 10 second dwell time
- X50 objective

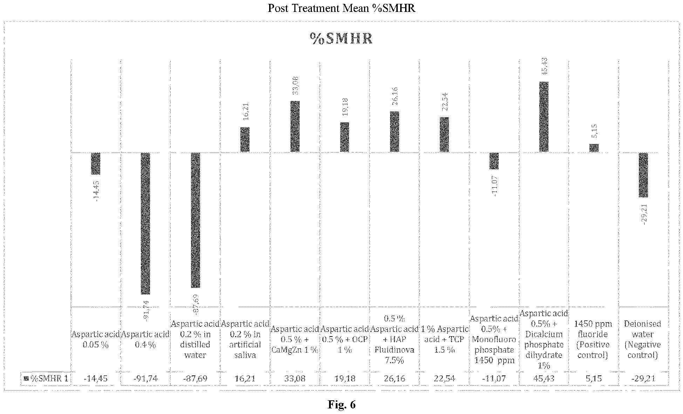

The schematic diagram in Figure 6, shows the approximate location of the indents. Spacings were approximate and dependent on size of indents and avoidance of surface scratches and other imperfections.

1.4 Enamel Block Demineralisation

A demineralisation solution was prepared that contained:

- 50 mM Acetic acid
- 2.2 mM Calcium nitrate
- 2.2 mM Potassium phosphate monobasic
- 0.1 ppm Sodium fluoride

The pH of the solution was adjusted to pH 4.5. All pH measurements were taken with a calibrated pH electrode (Serial Number: 13-620-631) and either an Accumet XI,250 ion meter (Asset Number: UK01200-LAB-15-002), or an Orion 720 ion meter (Asset Number: 4F064).

The enamel blocks in groups 1 to 7 were demineralised by placing the samples into a Sterilin containing 8mL of demineralisation solution per sample. All demineralisation exposures were performed for 60 minutes, in an oven set to 37°C for 60 minutes.

Following removal of the samples from the demineralisation solution, each enamel block was rinsed with deionised water for 1-2 minutes and returned to its original container.

The post demineralisation surface microhardness of the samples was then remeasured.

1.5 Removal of Outliers and Stratification

Specimens with "outlier" baseline/demineralised SMH values were removed from the study. 70 blocks were stratified into 7 treatment groups (n=10) to create groups with similar mean baseline SMH values.

2. Treatment

2.1 Test Product Treatment

The enamel samples were placed into their assigned test solutions for 16 hours at 37°C, whilst being agitated with a magnetic stirrer. Aquasil Putty was used to attach the 10 enamel blocks in each treatment group to a sample holder with the enamel surfaces of the samples exposed.

Following removal of the sample from the test product, each enamel block was rinsed with deionised water for 1-2 minutes and returned to their original containers. The enamel samples were sonicated for 10 minutes to remove any loose debris.

2.2 Data Management

The ability of the test formulations to repair the demineralised enamel samples after a single treatment was assessed by calculating the percentage of surface microhardness recovery values (%SMHR) achieved by each test product.

The formula for calculating the %SMHR values achieved by each treatment is shown below:

■

$$\% \ SMHR = 100 * [(SMH \ Post \ treatment - SMH \ Post\text{-}demineralisation) \ / \ (SMH \ Baseline - SMH \ Post\text{-}demineralisation)]$$

2.3 %SMHR Data

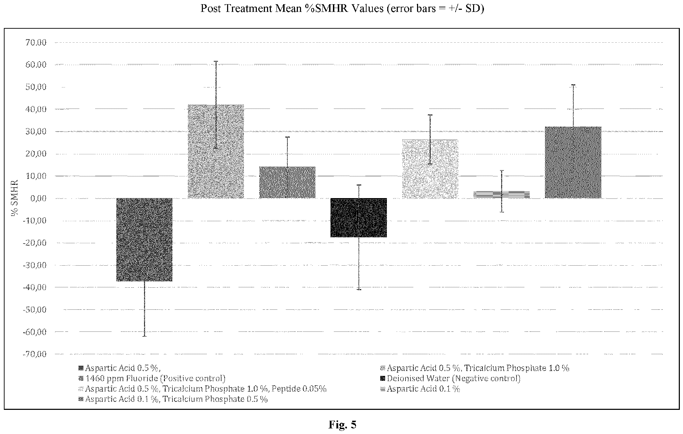

The mean %SMHR values achieved by the test products after a single treatment are shown in Table 6 and presented graphically in Figure 5.

Table 6. Post Treatment Mean %SMHR

| Treatments | Mean %SMHR | Grouping | | | | |
|---|---|---|---|---|---|---|
| Aspartic Acid 0.5 %, Tricalcium Phosphate 1.0 % | 42.03 | A | | | | |
| Aspartic Acid 0.1 %, Tricalcium Phosphate 0.5 % | 32.13 | A | B | | | |
| Aspartic Acid 0.5 %, Tricalcium Phosphate 1.0 %, Peptide 0.05% | 26.50 | A | B | C | | |
| 1450 ppm Fluoride (Positive control) | 14.12 | | B | C | | |
| Aspartic Acid 0.1 % | 3.19 | | | C | D | |
| Deionised Water (Negative control) | -17.52 | | | | D | E |
| Aspartic Acid 0.5 %, | -37.32 | | | | | E |

Means that do not share a letter are significantly different.

The test product containing Aspartic Acid 0.5 % & Tricalcium Phosphate 1.0 %, achieved the highest mean % SMHR value on this study (+42%).

Statistical analysis of the %SMHR data showed the test product containing Aspartic Acid 0.5 % & Tricalcium Phosphate 1.0 %, achieved a directionally higher %SMHR value than the following test products: Aspartic Acid 0.1 % & Tricalcium Phosphate 0.5 % and Aspartic Acid 0.5 % & Tricalcium Phosphate 1.0 % & Peptide 0.05 %.

Statistical analysis of the %SMHR data showed the test product containing Aspartic Acid 0.5 % & Tricalcium Phosphate 1.0 %, achieved a statistically significantly higher %SMHR value than the following test products: 1450 ppm Fluoride (Positive control), Aspartic Acid 0.1 %, Deionised Water (Negative control) and Aspartic Acid 0.5 %.

The test product containing Aspartic Acid 0.1 % & Tricalcium Phosphate 0.5 % and the test product containing Aspartic Acid 0.5 % & Tricalcium Phosphate 1.0 % & Peptide 0.05 % achieved the second and third highest mean %SMHR values on this study, with the test products achieving mean %SMHR values of 32 % and 27 % respectively.

Statistical analysis of the %SMHR data showed the test product containing Aspartic Acid 0.1 % & Tricalcium Phosphate 0.5 % and the test product containing Aspartic Acid 0.5 % & Tricalcium Phosphate 1.0 % & Peptide 0.05 % achieved a directionally higher %SMHR value than fluoride and a statistically significantly higher %SMHR value than the following test products: Aspartic Acid 0.1 %, Deionised Water (Negative control) and Aspartic Acid 0.5 %.

The test product containing fluoride, achieved the fourth highest mean %SMHR value on this study (+14 %).

Statistical analysis of the %SMHR data showed the test product containing fluoride, achieved a directionally higher %SMHR value than the test product containing Aspartic Acid 0.1 % and a statistically significantly higher %SMHR value than Deionised Water (Negative control) and Aspartic Acid 0.5 %).

Deionised water and the test product containing Aspartic Acid 0.5 % achieved the lowest mean %SMHR values on this study, with the test products achieving mean %SMHR values of -18 % and -37 % respectively.

Statistical analysis of the %SMHR data showed deionised water achieved a directionally lower %SMHR value than the test product containing Aspartic Acid 0.1 % and a statistically significantly lower %SMHR value than the following test products: Aspartic Acid 0.5 % & Tricalcium Phosphate 1.0 %, Aspartic Acid 0.1 % & Tricalcium Phosphate 0.5 %, Aspartic Acid 0.5 % & Tricalcium Phosphate 1.0 % & Peptide 0.05 % and Fluoride.

3. Conclusions

After a single overnight application, test products containing Aspartic acid 0,5% and Tricalcium Phosphate 1% achieved the highest mean %SMHR values on this study.

EXPERIMENT 3

An *In Vitro* Study to Compare the Ability of different calcium sources combined with aspartic acid Versus a Positive and Negative Control to Repair and Protect Demineralised hard tooth tissues Samples

1. Study Design

Enamel blocks were sectioned from bovine teeth, lapped plane-parallel, and highly polished. The baseline surface microhardness of the enamel samples was measured. The samples were then stratified between the treatment groups based on the baseline surface microhardness values.

The enamel samples were artificially demineralised by immersing them in a demineralisation solution for 60 minutes. Following demineralisation, the surface microhardness of the enamel samples was remeasured.

The enamel samples in each treatment group were treated with the assigned test products for 16 hours. Following treatment, the surface microhardness of each treated enamel sample was remeasured.

The percentage of surface microhardness recovery (%SMHR) achieved by the test products was calculated and used to compare the ability of the test products to repair the demineralised enamel samples.

1.1 Test Products

The test products detailed in Table 8 were assessed in this study.

Table 7. Test Product Codes

| Test Product Description | Test Product Code |
|---|---|
| Aspartic acid | No Batch I.D. |
| CaMgZn HAP Dry | No Batch I.D. |

(continued)

| Test Product Description | Test Product Code |
|---|---|
| Octacalcium Phosphate | No Batch I.D. |
| HAP Fluidinova | No Batch I.D. |
| Tricalcium Phosphate | No Batch I.D. CAS: 7758-87-4 |
| Dicalcium Phosphate Dihydrate Pure | Protolab<br>CAS: 7789-77-7<br>WE:616-542-1<br>Data waznosci: 03.2025<br>Seria: 0323 |
| Deionised Water (Negative control) | Scientific Laboratory Supplies, Product Code: CHE3876<br>Batch: 872466<br>Exp: 13/03/2024 |
| Sodium Fluoride (Positive Control) | Sigma, Product Code: 1003217431<br>Exp: 5/4/2027 |

The test products detailed in Table 1 were applied to the samples in the concentrations detailed in Table 8.

Table 8. Test Product Concentrations

| Treatment Group | Treatment |
|---|---|
| 1 | Aspartic acid 0.05 % |
| 2 | Aspartic acid 0.4 % |
| 4 | Aspartic acid 0,2% in artificial saliva |
| 4 | Aspartic acid 0.2 % in distilled water |
| 5 | Aspartic acid 0.5 % + CaMgZn 1 % |
| 6 | Aspartic acid 0.5 % + OCP 1 % |
| 7 | 0.5 % Aspartic acid + HAP Fluidinova 7.5 % |
| 8 | 1 % Aspartic acid + TCP 1.5 % |
| 8 | Aspartic acid 0.5% + Monofluorophosphate 1450 ppm |
| 10 | Aspartic acid 0.5% + Dicalcium phosphate dihydrate 1% |
| 11 | Fluoride (Positive control) 100 % Sodium Fluoride powder Prepared as 1450ppm F$^-$ in deionised water |
| 12 | Deionised water (Negative control) |

The pH of each solution was adjusted into the pH range of pH 6 to 6.5, using sodium hydroxide or dilute hydrochloric acid (Excluding the positive and negative control). All pH measurements were taken with a calibrated pH electrode (Serial Number: VT1 8048) and an Accumet XI,250 ion meter (Asset Number: UK01200-LAB-15-002).

1.2 Block Preparation

Approximately 130 enamel blocks (Measuring 4mm x 4mm) were sectioned from bovine incisors and were lapped plano-parallel (both sides) using a Logitech PM5 lapping machine (Asset number: 4F008).
The enamel surfaces of the samples were machine-polished with 1 micron and 0.3 micron aluminium oxide slurries, using a MetPrep Saphir 550 Polisher (Asset Number: UK01200-1010015-114).
One corner of each block was removed to facilitate consistent orientation of the samples on the surface microhardness machine. All prepared blocks were stored in individual Sterilins, on a layer of tissue paper dampened with 0.1% thymol.

1.3 Surface Microhardness (SMH) Measurements

The surface microhardness of each block was measured at the following time points:

- At baseline.
- After the demineralisation treatment.
- After overnight treatment.

The surface microhardness of the enamel samples was measured using a Buehler MicroMet 5103 (Asset Number: 4F101) and OmniMet MHT software.

The Intertek CRS method for measuring the surface microhardness of the samples at each time point has been provided below:

- Five Knoop indents
- 50 gram load force
- 10 second dwell time
- X50 objective

The schematic diagram in Figure 6, shows the approximate location of the indents. Spacings were approximate and dependent on the size of the indents and avoidance of surface scratches and other imperfections.

1.4 Removal of Outliers and Stratification

Specimens with "outlier" baseline SMH values were removed from the study. 120 blocks were stratified into 12 treatment groups (n=10) to create groups with similar mean baseline SMH values.

1.5 Enamel Block Demineralisation

A demineralisation solution was prepared that contained:

- 50 mM Acetic acid
- 2.2 mM Calcium nitrate
- 2.2 mM Potassium phosphate monobasic
- 0.1 ppm Sodium fluoride

The pH of the solution was adjusted to pH 4.5. All pH measurements were taken with a calibrated pH electrode (Serial Number: VT1 8048) and an Accumet XI,250 ion meter (Asset Number: UK01200-LAB-15-002.

The enamel blocks were demineralised by placing the samples into a Sterilin containing 8mL of demineralisation solution per sample. All demineralisation exposures were performed for 60 minutes, in an oven set to 37°C for 60 minutes.

Following the removal of the samples from the demineralisation solution, each enamel block was rinsed with deionised water for 1-2 minutes and returned to its original container. The post demineralisation surface microhardness of the samples was then remeasured.

2. Treatment

2.1 Test Product Treatment

The enamel samples were placed into their assigned test solutions for 16 hours at 37°C, whilst being agitated with a magnetic stirrer. Aquasil Putty was used to attach the 10 enamel blocks in each treatment group to a sample holder with the enamel surfaces of the samples exposed.

Following the removal of the samples from the test product, each enamel block was rinsed with deionised water for 1-2 minutes and sonicated for 10 minutes to remove any loose debris. The enamel samples were then returned to their original containers.

2.2 Data Management

The ability of the test formulations to repair the demineralised enamel samples after a single treatment was assessed by calculating the percentage of surface microhardness recovery values (%SMHR) achieved by each test product.

The formula for calculating the %SMHR values achieved by each treatment is shown below:

$$\% \text{ SMHR} = 100 * [(\text{SMH Post-treatment} - \text{SMH Post-demineralisation}) / (\text{SMH Baseline} - \text{SMH Post-demineralisation})]$$

All surface microhardness data was doubled data entered from the SMH reports, merged, and checked in accordance with Intertek CRS data management standard operating procedures, with data checks signed and scanned into the electronic study folder.

3. Results

The mean %SMHR values achieved by the test products after a single treatment are shown in Table 3 and presented graphically in Figure 2.

The p-value table provides a comprehensive guide to ranking the test products by highlighting statistically significant differences between the %SMHR values achieved by the different test solutions (Table 4).

Table 9. Post-Treatment Mean %SMHR

| Treatment | Mean %SMHR Value | StDev |
|---|---|---|
| 0.5 % Aspartic acid + HAP Fluidinova 7.5% | 26.16 | 13.91 |
| 1 % C4H7NO4 + TCP 1.5 % | 22.54 | 8.65 |
| 1450 ppm fluoride (Positive control) | 5.15 | 4.84 |
| Aspartic acid 0.05 % | -35.13 | 24.84 |
| Aspartic acid 0.2 % in distilled water | -87.69 | 47.76 |
| Aspartic acid 0,2% in artificial saliva | 16,21 | 8,35 |
| Aspartic acid 0.4 % | -91.74 | 27.01 |
| Aspartic acid 0.5 % + CaMgZn 1 % | 33.08 | 14.84 |
| Aspartic acid 0.5 % + OCP 1 % | 19.18 | 10.52 |
| Aspartic acid 0.5% + Dicalcium phosphate dihydrate 1% | 45.43 | 14.64 |
| Aspartic acid 0.5% + SMFP1450 ppm | -11.07 | 11.16 |
| Deionised water (Negative control) | -29.21 | 18.38 |

The lowest %SMHR values were achieved by the 0.4% and the 0.2% Aspartic acid solutions in distilled water, which achieved mean %SMHR values of -92% and -88% respectively.

Statistical analysis of the data showed the 0.4% and 0.2% Aspartic acid solutions in distilled water achieved statistically significantly lower %SMHR values than all other test solutions assessed in this study.

The data indicates the 0.4% and the 0.2% Aspartic acid solutions in distilled water continued to reduce the surface microhardness of the enamel samples during the overnight treatment.

The highest %SMHR value was achieved by the test solution containing 0.5% Aspartic acid and 1% Dicalcium phosphate dihydrate, which achieved a mean %SMHR value of 45%.

Statistical analysis of the study data showed the test solution containing 0.5% Aspartic acid and 1% Dicalcium phosphate dihydrate achieved a statistically significantly higher %SMHR value than the following test solutions: 1% Aspartic acid + 1.5% TCP, 1450 ppm fluoride (positive control), 0.4% Aspartic acid, 0.2% Aspartic acid, 0.05% Aspartic acid, Deionised water (negative control), Aspartic acid 0.5 % + OCP 1 % and Aspartic acid 0.5% + SMFP 1450 ppm.

Statistical analysis of the study data showed the test solution containing 0.5% Aspartic acid and 1% dicalcium phosphate dihydrate achieved a directionally higher %SMHR value than the test solution containing 0.5 % Aspartic acid + HAP Fluidinova 7.5% and the test solution containing Aspartic acid 0.5 % + CaMgZn 1 %.

The second highest %SMHR value was achieved by the test solution containing Aspartic acid 0.5 % + CaMgZn 1 %, which achieved a mean %SMHR value of 33%.

Statistical analysis of the study data showed the test solution containing Aspartic acid 0.5 % + CaMgZn 1 % achieved a statistically significantly higher %SMHR value than the following test solutions: 1450 ppm Fluoride (positive control), 0.4% Aspartic acid, 0.2% Aspartic acid, 0.05% Aspartic acid, Deionised water (negative control), Aspartic acid 0.5 % + OCP 1 %, and 0.5% Aspartic acid + SMFP 1450 ppm.

Statistical analysis of the study data showed the test solution containing Aspartic acid 0.5 % + CaMgZn 1 % achieved a directionally higher %SMHR value than the following test solutions: 1% Aspartic acid + 1.5% TCP, 0.5 % Aspartic acid + HAP Fluidinova 7.5%.

Statistical analysis of the study data showed the test solution containing Aspartic acid 0.5 % + CaMgZn 1 % achieved a directionally lower %SMHR value than the test solution containing 0.5% Aspartic acid and 1% Dicalcium phosphate dihydrate.

The third highest %SMHR value was achieved by the test solution containing 0.5 % Aspartic acid + HAP Fluidinova 7.5%, which achieved a mean %SMHR value of 26%.

Statistical analysis of the study data showed the test solution containing 0.5 % Aspartic acid + HAP Fluidinova 7.5%, Aspartic acid 0,2% in artificial saliva, Aspartic acid 0,05% in artificial saliva achieved a statistically significantly higher %SMHR value than the following test solutions: 1450 ppm Fluoride (positive control), 0.4% Aspartic acid, 0.2% Aspartic acid, 0.05% Aspartic acid, Deionised water (negative control) and 0.5% Aspartic acid + SMFP 1450 ppm.

Statistical analysis of the study data showed the test solution containing 0.5 % Aspartic acid + HAP Fluidinova 7.5%, Aspartic acid 0,2% in artificial saliva, Aspartic acid 0,05% in artificial saliva achieved a directionally higher %SMHR value than the following test solutions: 1 % Aspartic acid + TCP 1.5% and Aspartic acid 0.5 % + OCP 1 %.

Statistical analysis of the study data showed the test solution containing 0.5 % Aspartic acid + HAP Fluidinova 7.5%, Aspartic acid 0,2% in artificial saliva, Aspartic acid 0,05% in artificial saliva achieved a directionally lower %SMHR value than the following test solutions: Aspartic acid 0.5% + Dicalcium phosphate dihydrate 1% and Aspartic acid 0.5 % + CaMgZn 1 %.

### Conclusions

After a single overnight treatment, the 0.4% and 0.2% Aspartic acid in distilled water solutions continued reducing the enamel samples' surface microhardness.

After a single overnight treatment, the 0.2% Aspartic acid in artificial saliva solution increase microhardness of tested samples.

Overall best performing test solutions within the study were: Aspartic acid 0.5% + Dicalcium phosphate dihydrate 1%, Aspartic acid 0.5 % + CaMgZn 1 % and 0.5 % Aspartic acid + HAP Fluidinova 7.5%.

Conclusion

Table 10. Summary of 3 experiments in comparison with negative and positive controls

| System | Comparison |
|---|---|
| Aspartic acid 0.5% + Dicalcium phosphate dihydrate 1% | for 117 units better than fluoride |
| Octacalciumphosphate (OCP) | for 95 units better than fluoride |
| Aspartic Acid 0.5 %, Tricalcium Phosphate 1.0 % | for 88 units better than fluoride |
| Aspartic acid 0.5 % + CaMgZn 1 % | for 81 units better than fluoride |
| Aspartic Acid 0.1 %, Tricalcium Phosphate 0.5 % | for 56 units better than fluoride |
| 0.5 % Aspartic acid + 1% NanoXim*CarePaste Hydroxyapatite (nano) | for 61 units better than fluoride |
| 1 % Aspartic acid + TCP 1.5 % | for 51 units better than fluoride |
| Aspartic acid 0.5 % + OCP 1 % | for 41 units better than fluoride |
| Aspartic acid 0,2% in artifical saliva | for 25 units better than fluoride |
| Aspartic acid 0,1% in artifical saliva | for 2 units better than fluoride |
| Fluoride (Positive control) | 100 units |
| 1% CaMgZnAHAp solution | for 77 units better than negative control |
| 1% CaMgZnAHAp dry | for 66 units better than negative control |
| Aspartic acid 0,1% | for 65 units better than negative control |

(continued)

| System | Comparison |
|---|---|
| 1% NanoXim*CarePaste Hydroxyapatite (nano) | for 62 units better than negative control |
| Aspartic acid 0.5% + Monofluorophosphate 1450 ppm | for 52 units better than negative control |
| 1% Tricalcium phopshate | for 44 units better than negative control |
| 1% CaHAP dry | for 43 units better than negative control |
| 1% CaHAP solution | for 24 units better than negative control |
| 0,05% Aspartic acid | for 17 units better than negative control |
| Water (Negative control) | 0 units |
| Aspartic acid 0,2% in distilled water | for 70 units worser than negative control |
| Aspartic acid 0,4% in distilled water | for 82 units worser than negative control |
| Aspartic acid 0,5% in distilled water | for 90 units better than negative control |

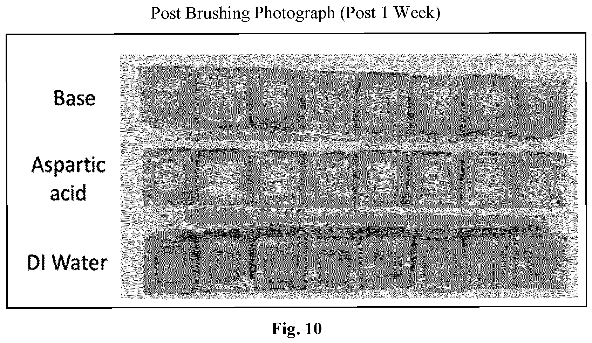

Based on the comparative results of all systems from experiments 1-3 (Table 11, Figure 10), the following conclusions can be drawn:

- Aspartic acid at a concentration of 0.05%-0.2% works as a remineralizing agent in the presence of saliva, which is the saline solution when using aspartic acid in Oral care.

- Aspartic acid in concentrations above 0.2% demineralizes enamel without the presence of saliva components, which are sources of phosphorus and calcium.

- Aspartic acid in concentrations of 0.1% to 1% is a remineralizing agent in the presence of calcium sources.

- Aspartic acid is the effective solution for remineralization of hard tissues in the tooth in the concentration and combinations above.

EXPERIMENT 4

Study Title: *In Vitro* Study to Compare the Whitening Efficacy of Two Toothpastes (Base toothpaste vs Tootphaste with Aspartic acid) Versus a Negative Control

Analytical Objectives

Use acellular, stained bovine enamel blocks to compare the ability of 2 toothpaste formulations versus a negative control to remove stains after the equivalent of one week's worth of brushing for a single tooth.
To use the total colour change (Delta E) values to compare the ability of 2 toothpaste formulations versus a negative control to remove stains after the equivalent of one week's worth of brushing for a single tooth.

Overview

This test was carried out using blocks of bovine enamel that were set into cuvettes using dental acrylic.
The enamel surfaces of the samples were lightly etched to facilitate stain uptake and the enamel samples were stained using a staining solution containing tea, coffee, mucin and ferric chloride. The post stain colour of the enamel samples was measured with a calibrated Konica Minolta CM-700d Spectrophotometer.
Each stained sample was brushed with a V8 brushing machine and the assigned test product slurry for the equivalent of 1 week's worth of brushing for a single tooth. The post treatment colour of the enamel samples was measured after the equivalents of 1 week's worth of brushing using a calibrated Konica Minolta CM-700d Spectrophotometer.
The changes in each colour parameter (L*, a*, b*) were calculated and used to determine delta E, which is a measure of the total level of stain removal. The delta E values achieved by each test product were statistically compared, with higher delta E values being indicative of greater whitening efficacy.

Table 11. Treatments

| Product Information |
|---|
| Toothpaste with aspartic acid |
| Base toothpaste without whitening active ingredients |
| Deionised Water |

Study Preparation

Enamel Block Preparation

The samples used on this study were taken from Intertek stocks and were originally prepared as described below.

Forty blocks were prepared using a dental abrader to obtain labial enamel specimens from bovine incisors, with 7mm x 7mm dimensions. The dentine surfaces of the samples were grooved and painted with clear nail varnish to prevent staining of the dentine layer.

The enamel blocks were embedded in polystyrene cuvettes using methacrylate resin so that only the enamel surfaces of the samples were exposed to the staining solution. All samples were polished using 400 and 600 grit, silicon carbide paper.

All samples were visually inspected to ensure the enamel surfaces were sufficiently polished and free from defects. Finally, the methacrylate resin within each block was drilled to facilitate the attachment of the samples to the staining rig.

Staining of the Enamel Blocks

The enamel surfaces of the samples were lightly etched to facilitate stain uptake. Blocks were etched by immersing them in 1% HCl (1 minute), followed by a saturated sodium carbonate (30 seconds) and 1% phytic acid (1 minute). The blocks were dabbed dry between each etching stage and rinsed after the final immersion in phytic acid.

A staining solution was prepared that contained tryptone soy broth (TSB), PG Tips instant tea, Nescafe instant coffee, mucin type II, ferric chloride, and deionised water.

The enamel samples were stained for four days using a specially constructed rig to cycle the samples in and out of stain solution that was heated to 50oC.

Staining Colour Measurements & Photographs

The post stain colour (L*, a*, b*) of the enamel samples was measured with a calibrated CM-700d spectrophotometer (UK-01200-LAB-13-220).

A photograph of the samples in each treatment group after staining was captured with a digital camera.

Main Study

Treatment

Toothpaste slurries were prepared in the ratio of 1 gram of toothpaste to 1.6 grams of RO water. All toothpaste slurries were prepared immediately prior to being applied to the samples and were homogenised using an electronic stirrer. For each brushing, 90ml of toothpaste slurry was added to each well of the V8 brushing machine.

All brushing treatments were performed with 90ml of the assigned toothpaste slurry and a V8 brushing machine. A single toothpaste slurry was used to brush each sample for the equivalent of 1 week's worth of brushing for a single tooth.

The following settings were used for all brushing treatments:

150 gram load.
100 rubs per minute.

The height of the individual samples was adjusted within the V8 brushing machine to ensure a consistent height of the samples during brushing.

Brushing time was equivalent to one week's worth of brushing for a single tooth. The calculation used to determine the length of brushing has been explained below:

It is commonly recommended for consumers to brush their teeth twice daily, for a duration of 2 minutes per brushing (240 seconds). When this routine is replicated for 7 days, the brushing time totals 28 minutes (1680 seconds). The maximum number of teeth in a human mouth is 32. One week's worth of brushing for a single tooth can be calculated by dividing 1680 seconds by 32 teeth to give a brushing time of 52.5 seconds.

Post Treatment Colour Measurements & Photographs

The colour (L*, a*, b*) of the enamel samples after the equivalent of 1 week's worth of brushing for a single tooth were measured with a calibrated CM-700d spectrophotometer.
For all colour measurements, the following settings were selected:

- Aperature: SAV (3 mm aperture)
- Gloss: SCI
- Illuminant: D65
- Observer: 1964 10o
- Auto Average: 3

Colour measurements were performed with the enamel samples in a hydrated state, with each enamel sample dabbed dry and four colour measurements performed at a 90-degree angle from the previous measurement.
A photograph of the samples in each treatment group was captured with a digital camera after the equivalent of 1 week's worth of brushing for a single tooth. The photographs were not intended for analysis and were provided for illustration purposes only. A photograph of the samples at the post-brushing time point has been included in this report (Figures 4).

Results

Delta E Results

Table 13 shows the mean Delta E values achieved by each toothpaste formulation and the negative control after the equivalent of 1 week's worth of brushing for a single tooth. For clarity, the Delta E data is also presented graphically in Figure 11.
Table 14 highlights statistically significant differences between the Delta E values achieved by the test products and negative control after the equivalent of 1 week's worth of brushing for a single tooth.

Table 12. Mean Delta E Changes (Post 1 Week)

| Treatment | Mean Change in Delta E | StDev |
|---|---|---|
| Paste 1 (The base toothpaste without whitening agent) | 7.58 | 0.94 |
| Paste 2 (The base toothpaste with aspartic acid) | 9.12 | 1.32 |
| Negative control (DI Water) | 0.34 | 0.19 |

Table 13. Tukey Pairwise Comparisons for Delta E Values (Post 1 Week)

| Treatment | N | Mean | Grouping | | |
|---|---|---|---|---|---|
| Paste | 8 | 9.12 | A | | |
| Paste 4 | 8 | 7.57 | A | B | |
| DI Water | 8 | 0.34 | | | C |
| *Means that do not share a letter are significantly different. | | | | | |

The toothpaste formulations were all effective at removing stains, with the two toothpaste formulations all achieving statistically significantly higher levels of stain removal (Delta E) than the negative control (Table 14).
After the equivalent of 1 week's worth of brushing for a single tooth, the toothpaste formulation coded Toothpaste with aspartic acid achieved the highest level of stain removal, achieving a mean delta E value 9 units.
Statistical analysis of the data showed the differences between the levels of whitening achieved by the two toothpaste formulations were statistically significant.

Photographs

A photograph of the enamel samples before staining is shown in Figure 12, whilst Figure 13 shows a photograph of the enamel samples after staining.

Conclusions

The toothpaste formulations were effective at removing stains, with the two toothpaste formulations all achieving statistically significantly higher levels of stain removal (Delta E) than the negative control.

The toothpaste coded Toothpaste with aspartic acid achieved the highest level of stain removal in the study, achieving a directionally higher delta E value than the comparator toothpaste.

LITERATURE

1) Esiev R.K., Zakaeva R.Sh., Isaeva S.E. INFLUENCE OF FOOD ACIDITY ON THE PH OF SALIVA AND MOUTH CAVITY // International Student Scientific Bulletin. - 2015. - No. 3-4.;

2) Tanner ACR, Kressirer CA, Rothmiller S, Johansson I, Chalmers NI. 2018. The caries microbiome: implications for reversing dysbiosis. Adv Dent Res. 29(1):78-85.

3) ten Cate, O., Chen, H. C., Hoff, R. G., Peters, H., Bok, H., & van der Schaaf, M. (2015). Curriculum development for the workplace using Entrustable Professional Activities (EPAs): AMEE Guide No. 99. Medical Teacher, 37(11), 983-1002

4) Reynolds EC, Cai F, Cochrane NJ, et al. Fluoride and casein phosphopeptide-amorphous calcium phosphate. J Dent Res 2008; 87: 344- 348.

5) Esiev R.K., Zakaeva R.Sh., Isaeva S.E. INFLUENCE OF FOOD ACIDITY ON THE PH OF SALIVA AND MOUTH CAVITY // International Student Scientific Bulletin. - 2015. - No. 3-4. ;

6) Weissman-Fogel, I., Sprecher, E. and Pud, D., 2008. Effects of catastrophizing on pain perception and pain modulation. Experimental brain research, 186, pp.79-85.

7) Ruan, Qichao & Moradian-Oldak, Janet. (2015). Amelogenin and Enamel Biomimetics. Journal of Materials Chemistry B. 3. 3112. 10.1039/C5TB00163C.

8) Alkilzy M, Tarabaih A, Santamaria RM, Splieth CH. Self-assembling Peptide P11-4 and Fluoride for Regenerating Enamel. J Dent Res. 2018 Feb;97(2):148-154. doi: 10.1177/0022034517730531. Epub 2017 Sep 11. PMID: 28892645; PMCID: PMC6429572.

9) Moradian-Oldak J: Protein-Mediated Enamel Mineralization. Front Biosci (Landmark Ed) 2012; 17 (6):1996-2023. DOI: 10.2741/4034

10) Therapeutic Management of Demineralized Dentin Surfaces Using a Mineralizing Adhesive To Seal and Mineralize Dentin, Dentinal Tubules, and Odontoblast Processes. Ying Shi, Dongni Shen, Haiyan Zheng, Zhifang Wu, Changyu Shao, Leiqing Zhang, Haihua Pan, Ruikang Tang, and Baiping Fu. *ACS Biomaterials Science & Engineering* 2019 5 (10), 5481-5488. DOI: 10.1021/acsbiomaterials.9b00619

11) Hou, A., Luo, J., Zhang, M. et al. Two-in-one strategy: a remineralizing and anti-adhesive coating against demineralized enamel. Int J Oral Sci 12, 27 (2020). https://doi.org/10.1038/s41368-020-00097-y

12) Relationships between Structure and Properties of Poly(aspartic acid)s. Takeshi Nakato, Masako Yoshitake, Koshi Matsubara, Masayuki Tomida, and Toyoji Kakuchi. Macromolecules 1998 31 (7), 2107-2113. DOI: 10.1021/ma971629y

13) Polymer-Induced Liquid Precursor (PILP) remineralization of artificial and natural dentin carious lesions evaluated by nanoindentation and microcomputed tomography. Elham Babaie, Marg6t Bacino, Joel White, Hamid Nurrohman, Grayson W. Marshall, Kuniko Saeki, Stefan Habelitz. June 2021. https://doi.org/10.1016/j.jdent.2021.103659

14) Ren Q, Ding L, Li Z, Wang X, Wang K, Han S, Li W, Zhou X, Zhang L. Chitosan hydrogel containing amelogenin-derived peptide: Inhibition of cariogenic bacteria and promotion of remineralization of initial caries lesions. Arch Oral Biol. 2019 Apr;100:42-48. doi: 10.1016/j.archoralbio.2019.02.004. Epub 2019 Feb 10. PMID: 30782523.

15) Dogan, Sami & Fong, Hanson & Yucesoy, Deniz & Cousin, Timothee & Gresswell, Carolyn & Dag, Sefa & Huang, Greg & Sarikaya, Mehmet. (2018). Biomimetic Tooth Repair: Amelogenin-Derived Peptide Enables in Vitro Remineralization of Human Enamel. ACS Biomaterials Science & Engineering. 4. 10.1021/acsbiomaterials.7b00959.

16) H. Tong, W. Ma, L. Wang, P. Wan, J. Hu, L. Cao. Control over the crystal phase, shape, size and aggregation of calcium carbonate via a L-aspartic acid inducing process. Biomaterials, 25 (2004), pp. 3923-3929, 10.1016/j.biomaterials.2003.10.038

**EP 4 548 907 A1**

17) Golovanenko A.L. A review of remineralizing drugs used for the prevention and treatment of initial enamel caries / A.L. Golovanenko // Pacific Scientific Journal. - 2018. - No. 4 (74). - P.37-43

18) Fernando, J.R., Walker, G.D., Park, T.KS. et al. Comparison of calcium-based technologies to remineralise enamel subsurface lesions using microradiography and microhardness. Sci Rep 12, 9888 (2022). https://doi.org/ 10.1038/s41598-022-13905-8

19) Reynolds, E. (2008), Calcium phosphate-based remineralization systems: scientific evidence?. Australian Dental Journal, 53: 268-273. https://doi.org/10.1111/j.1834-7819.2008.00061.

## Claims

1. A composition comprising

   (A) 0.05 wt.% to 1.00 wt.% aspartic acid, and
   (B) a calcium source.

2. The composition of claim 1, wherein said composition comprises the aspartic acid as per the following wt.% sub-ranges in said composition: 0.1 - 1.0, 0.2 - 0.9, 0.3 - 0.8, 0.4 - 0.7, 0.5 - 0.6, and combinations thereof.

3. The composition of any of the preceding claims, wherein said calcium source comprises particles with crystalline or amorphous structure and a particle size from 0.001 $\mu$m to 100 $\mu$m and/or wherein said composition comprises said calcium source as per the following wt.% ranges in said composition: 0.1 - 15.0, 0.2 - 14.0, 0.3 - 13.0, 0.4 - 12.0, 0.5 - 11.0, 0.6 - 10.0, 0.7 - 9.0, 0.8 - 8.0, 0.9 - 7.0, 1.0 - 6.0, 1.5 - 5.0, 2.0 - 4.0, 2.5 - 3.5, 3.0 - 4.0, and combinations thereof..

4. The composition of claim 1, wherein said composition comprises the following wt.% in said composition: 0.10 - 1.00 aspartic acid and 0.1 - 15.0 calcium source, preferably 0.30 - 0.80 aspartic acid and 0.5 - 2.0 calcium source, further preferably 0.40 - 0.60 aspartic acid and 0.8 - 1.5 calcium source.

5. The composition of any of the preceding claims, wherein said calcium source is selected from the group consisting of: calcium comprising hydroxyapatite, calcium chitosan, calcium sodium fluorophosphosilicate, calcium acrylate, calcium citrate, calcium sorbate, calcium pantothenat, calcium gluconate, calcium aluminum borosilicate, calcium sodium phosphosilicate, calcium polygamma-glutamate, calcium carbonate, calcium monofluorophosphate, calcium silicate, calcium oxide, calcium ascorbate, calcium glycerophosphate, calcium peroxide, calcium lactate, dicalcium phosphate dihydrate, dicalcium phosphate, calcite, hydroxyapatite, potassium calcium hydroxyapatite, octacalcium phosphate, amorphous calcium phosphate, $\alpha$-tricalciumphosphate, $\beta$-tricalciumphosphate, dicalcium phosphate anhydrate, monocalcium phosphate monohydrate, monocalcium phosphate anhydrate, preferably tricalcium phosphate.

6. The composition of any of the preceding claims, wherein said aspartic acid

   - is selected from L-aspartic acid, D-aspartic acid, and DAL-aspartic acid; and/or
   - has a CAS number selected from 56-84-8, 1783-96-6 or 617-45-8.

7. The composition of any of the preceding claims, wherein said composition has a pH of 4.0 to 8.5, preferably selected from the following pH sub-ranges: 4.5-8.0, 5.0-7.5, 5.5-7.0, 6.0-6.5, and combinations thereof.

8. The composition of any of the preceding claims, said composition not comprising fluoride and/or polyaspartic acid.

9. The composition of any of the preceding claims, wherein said composition

   - is an oral care composition or oral care product, preferably selected from the group consisting of: a mouthwash, a toothpaste, an oral foam, an oral gel, an oral teeth stripe or tooth cover, and/or
   - is a formulation selected from the following: film, aerosol, suspension, solution, tincture, cream, paste, lotion, ointment, gel, powder, granulate.

10. Use of the composition, oral care composition or oral care product of any of the preceding claims for non-medical and/or aesthetic use, wherein said use is preferably selected from the following group:

- for remineralization of teeth;
- for teeth and/or oral cavity cleaning;
- for preventing growth of bacteria in the oral cavity;
- for preventing destruction and/or for restoration of tooth enamel and dentin;
- for desensitizing of teeth;
- for teeth brushing.

11. Pharmaceutical preparation comprising the composition of any of claims 1-9.

12. The composition of any of claims 1-9 for use as a medicament.

13. The composition of any of claims 1-9 for use in the remineralization of teeth in a subject and/or for preventing destruction of a subject's teeth enamel, dentin or cementum.

14. The composition for use of any of claims 1-9 for use in the treatment of an oral disease in a subject, wherein said oral disease is selected from the group consisting of: caries, periodontitis, fluorosis, hyperesthesia, gingivitis; stomatitis, halitosis, sensitive calculus.

15. The composition for use of claim 13 or claim 14, wherein said subject is suffering from or at risk of fluorosis or fluoride intoxication and/or wherein said subject has undergone prior to said treatment teeth bleaching.

Visualization of the molecular structure of aspartic acid and asparagine demonstrating reaction groups

**Fig. 1**

Modified sample holder

**Fig. 2**

Mean Percentage Surface microhardness Recoveries (%SMHR)

Fig. 3

Schematic Representation of SMH Indent Placement

Fig. 4

Post Treatment Mean %SMHR Values (error bars = +/- SD)

**Fig. 5**

Post Treatment Mean %SMHR

## %SMHR

| | Aspartic acid 0.05 % | Aspartic acid 0.4 % | Aspartic acid 0.2 % in distilled water | Aspartic acid 0.2 % in artificial saliva | Aspartic acid 0.5 % + CaMgZn 1 % | Aspartic acid 0.5 % + OCP 1 % | Aspartic acid + HAP Fluidinova 7.5% | 0.5 % 1 % Aspartic acid + TCP 1.5 % | Aspartic acid 0.5% + Monofluoro phosphate 1450 ppm | Aspartic acid 0.5% + Dicalcium phosphate dihydrate 1% | 1450 ppm fluoride (Positive control) | Deionised water (Negative control) |
|---|---|---|---|---|---|---|---|---|---|---|---|---|
| %SMHR 1 | -14,45 | -91,74 | -87,69 | 16,21 | 33,08 | 19,18 | 26,16 | 22,54 | -11,07 | 45,43 | 5,15 | -29,21 |

Fig. 6

27

Comparative table of studies in Experiments 1-3 regarding positive (fluorine) and negative (deionized water) control

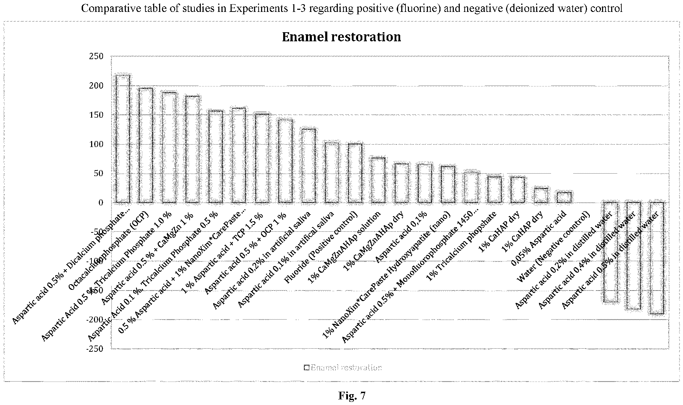

**Fig. 7**

Mean Delta E Change (Post 1 Week)

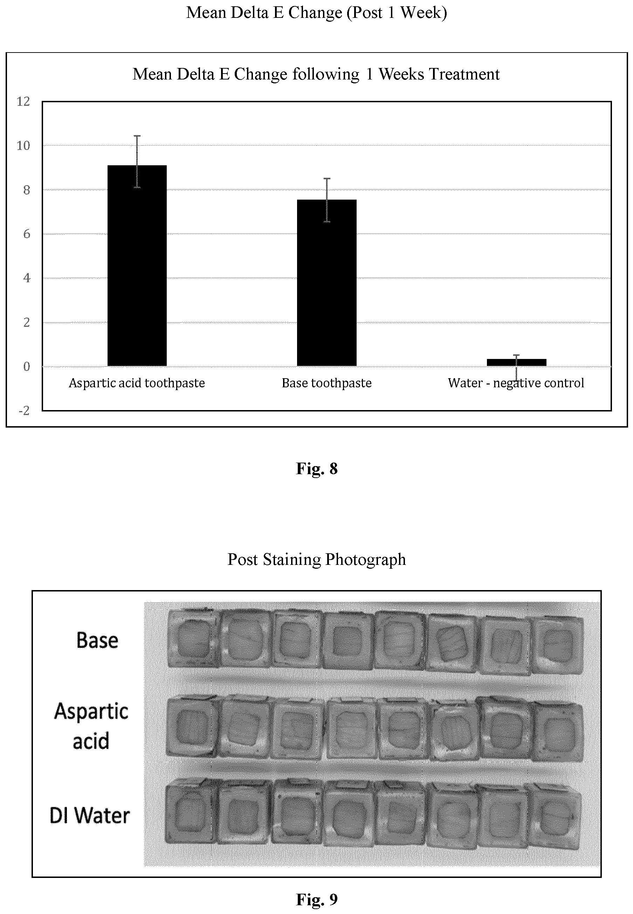

Fig. 8

Post Staining Photograph

Fig. 9

Post Brushing Photograph (Post 1 Week)

Base

Aspartic
acid

DI Water

**Fig. 10**

Europäisches
Patentamt

European
Patent Office

Office européen
des brevets

# EUROPEAN SEARCH REPORT

**Application Number**

**EP 23 20 7262**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 152 836 A2 (BLENDAX WERKE SCHNEIDER CO [DE]) 28 August 1985 (1985-08-28) * page 16; example 3 * * page 17; examples 7, 8 * * claims 1, 5 * | 1,2,5-7, 9-15 | INV. A61K8/19 A61K8/44 A61Q11/00 |
| X | DATABASE WPI Week 201728 Thomson Scientific, London, GB; AN 2017-15363Q XP002811238, & CN 106 420 437 A (ZHANG Y) 22 February 2017 (2017-02-22) * WPI abstract * | 1-4,6-15 | |
| X | WO 2020/263120 A1 (OBSHCHESTVO S OGRANICHENNOY OTVETSTVENNOSTYU SPLAT GLOBAL [RU]) 30 December 2020 (2020-12-30) * claims 1-17 * * paragraphs [0088] – [0089] * * paragraph [0097]; example 1 * | 1-15 | |
| X | DATABASE GNPD [Online] MINTEL; 10 February 2023 (2023-02-10), anonymous: "Cooling Mint Flavoured Amino Acid Gum Care Toothpaste", XP093142417, Database accession no. 10561774 * ingredients list; the whole document * | 1-15 | TECHNICAL FIELDS SEARCHED (IPC) A61K A61Q |
| X | WO 2013/034421 A2 (UNILEVER NV [NL]; UNILEVER PLC [GB] ET AL.) 14 March 2013 (2013-03-14) * claims 1, 12, 20, 21; example 4 * | 1-15 | |

-/--

The present search report has been drawn up for all claims

| Place of search | Date of completion of the search | Examiner |
|---|---|---|
| Munich | 22 March 2024 | Briand, Benoit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number

EP 23 20 7262

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | TONG H ET AL: "Control over the crystal phase, shape, size and aggregation of calcium carbonate via a l-aspartic acid inducing process", BIOMATERIALS, ELSEVIER, AMSTERDAM, NL, vol. 25, no. 17, 1 August 2004 (2004-08-01), pages 3923-3929, XP004496122, ISSN: 0142-9612, DOI: 10.1016/J.BIOMATERIALS.2003.10.038 * the whole document * | 1-15 | |

-----

TECHNICAL FIELDS SEARCHED (IPC)

The present search report has been drawn up for all claims

| Place of search | Date of completion of the search | Examiner |
|---|---|---|
| Munich | 22 March 2024 | Briand, Benoit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 23 20 7262

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2024

| Patent document cited in search report | | Publication date | Patent family member(s) | | Publication date |
|---|---|---|---|---|---|
| EP 0152836 | A2 | 28-08-1985 | CA | 1254836 A | 30-05-1989 |
| | | | EP | 0152836 A2 | 28-08-1985 |
| | | | ES | 8604417 A1 | 01-02-1986 |
| | | | US | 4622220 A | 11-11-1986 |
| | | | US | 4719100 A | 12-01-1988 |
| CN 106420437 | A | 22-02-2017 | NONE | | |
| WO 2020263120 | A1 | 30-12-2020 | RU | 2722306 C1 | 28-05-2020 |
| | | | WO | 2020263120 A1 | 30-12-2020 |
| WO 2013034421 | A2 | 14-03-2013 | BR | 112014004760 A2 | 28-03-2017 |
| | | | CN | 103764102 A | 30-04-2014 |
| | | | EP | 2753292 A2 | 16-07-2014 |
| | | | WO | 2013034421 A2 | 14-03-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *CHEMICAL ABSTRACTS*, 56-84-8
- *CHEMICAL ABSTRACTS*, 617-45-8
- *CHEMICAL ABSTRACTS*, 7758-87-4
- *CHEMICAL ABSTRACTS*, 7789-77-7
- **ESIEV R.K.** ; **ZAKAEVA R.SH.** ; **ISAEVA S.E.** INFLUENCE OF FOOD ACIDITY ON THE PH OF SALIVA AND MOUTH CAVITY. *International Student Scientific Bulletin*, 2015 (3-4)
- **TANNER ACR** ; **KRESSIRER CA** ; **ROTHMILLER S** ; **JOHANSSON I** ; **CHALMERS NI**. The caries microbiome: implications for reversing dysbiosis.. *Adv Dent Res.*, 2018, vol. 29 (1), 78-85
- **TEN CATE, O.** ; **CHEN, H. C.** ; **HOFF, R. G** ; **PETERS, H.** ; **BOK, H.** ; **VAN DER SCHAAF, M.** Curriculum development for the workplace using Entrustable Professional Activities (EPAs): AMEE Guide No. 99. *Medical Teacher*, 2015, vol. 37 (11), 983-1002
- **REYNOLDS EC** ; **CAI F** ; **COCHRANE NJ et al.** Fluoride and casein phosphopeptide-amorphous calcium phosphate.. *J Dent Res*, 2008, vol. 87, 344-348
- **ESIEV R.K.** ; **ZAKAEVA R.SH.** ; **ISAEVA S.E.** INFLUENCE OF FOOD ACIDITY ON THE PH OF SALIVA AND MOUTH CAVITY. *International Student Scientific Bulletin*, 2015 (3-4)
- **WEISSMAN-FOGEL, I.** ; **SPRECHER, E.** ; **PUD, D.** Effects of catastrophizing on pain perception and pain modulation. *Experimental brain research*, 2008, vol. 186, 79-85
- **RUAN, QICHAO** ; **MORADIAN-OLDAK, JANET.** Amelogenin and Enamel Biomimetics.. *Journal of Materials Chemistry B.*, 2015, vol. 3, 3112
- **ALKILZY M** ; **TARABAIH A** ; **SANTAMARIA RM** ; **SPLIETH CH**. Self-assembling Peptide P11-4 and Fluoride for Regenerating Enamel.. *J Dent Res.*, 11 September 2017, vol. 97 (2), 148-154
- **MORADIAN-OLDAK J**. Protein-Mediated Enamel Mineralization. *Front Biosci (Landmark Ed)*, 2012, vol. 17 (6), 1996-2023
- **HOU, A.** ; **LUO, J.** ; **ZHANG, M. et al.** Two-in-one strategy: a remineralizing and anti-adhesive coating against demineralized enamel. *Int J Oral Sci*, 2020, vol. 12, 27, https://doi.org/10.1038/s41368-020-00097-y
- **TAKESHI NAKATO** ; **MASAKO YOSHITAKE** ; **KOSHI MATSUBARA** ; **MASAYUKI TOMIDA** ; **TOYOJI KAKUCHI**. Relationships between Structure and Properties of Poly(aspartic acid)s.. *Macromolecules*, 1998, vol. 31 (7), 2107-2113
- **ELHAM BABAIE** ; **MARG6T BACINO** ; **JOEL WHITE** ; **HAMID NURROHMAN** ; **GRAYSON W. MARSHALL** ; **KUNIKO SAEKI** ; **STEFAN HABELITZ.** *Polymer-Induced Liquid Precursor (PILP) remineralization of artificial and natural dentin carious lesions evaluated by nanoindentation and micro-computed tomography.*, June 2021, https://doi.org/10.1016/j.jdent.2021.103659
- **REN Q** ; **DING L** ; **LI Z** ; **WANG X** ; **WANG K** ; **HAN S** ; **LI W** ; **ZHOU X** ; **ZHANG L**. Chitosan hydrogel containing amelogenin-derived peptide: Inhibition of cariogenic bacteria and promotion of remineralization of initial caries lesions. *Arch Oral Biol.*, 10 February 2019, vol. 100, 42-48
- **DOGAN, SAMI** ; **FONG, HANSON** ; **YUCESOY, DENIZ** ; **COUSIN, TIMOTHEE** ; **GRESSWELL, CAROLYN** ; **DAG, SEFA** ; **HUANG, GREG** ; **SARIKAYA, MEHMET.** Biomimetic Tooth Repair: Amelogenin-Derived Peptide Enables in Vitro Remineralization of Human Enamel. *ACS Biomaterials Science & Engineering*, 2018, vol. 4
- **H. TONG** ; **W. MA** ; **L. WANG** ; **P. WAN** ; **J. HU** ; **L. CAO.** Control over the crystal phase, shape, size and aggregation of calcium carbonate via a L-aspartic acid inducing process.. *Biomaterials*, 2004, vol. 25, 3923-3929
- **GOLOVANENKO A.L.** ; **A.L. GOLOVANENKO**. A review of remineralizing drugs used for the prevention and treatment of initial enamel caries. *Pacific Scientific Journal*, 2018, vol. 74 (4), 37-43
- **FERNANDO, J.R.** ; **WALKER, G.D.** ; **PARK, T.KS. et al.** Comparison of calcium-based technologies to remineralise enamel subsurface lesions using microradiography and microhardness. *Sci Rep*, 2022, vol. 12, 9888, https://doi.org/10.1038/s41598-022-13905-8
- **REYNOLDS, E**. Calcium phosphate-based remineralization systems: scientific evidence?.. *Australian Dental Journal*, 2008, vol. 53, 268-273, https://doi.org/10.1111/j.1834-7819.2008.00061